# FASCICULE DE BREVET EUROPEEN

(11) **EP 0 685 210 B1**
(45) Date de publication et mention de la délivrance du brevet: **17.11.1999**
(21) Numéro de dépôt: 95401018.7
(22) Date de dépôt: 03.05.1995
(51) Int. Cl.: A61F 2/38, A61B 17/14

(54) **Implant trochléen pour prothèse fémoro-patellaire et son instrumentation de pose**
Femorales kondiläres und patellares Implantat, und chirurgisches Instrument für die Implantierung
Femoral condylar and patellar implantable prosthesis and implantation instrumentation

(30) Priorité: 13.05.1994 FR 9405888
(43) Date de publication de la demande: 06.12.1995
(73) Titulaire: SMITH & NEPHEW RICHARDS FRANCE, 91781 Wissous Cédex (FR)
(72) Inventeur: Hummer, Jacques, F-54000 Nancy (FR); Dive, Michel, F-13000 Marseille (FR); Laurençon, Michel, F-69006 Lyon (FR); Clauze, Jacques, F-64300 Baigts de Bearn (FR)
(74) Mandataire: Ramey, Daniel

(56) Documents cités:
- EP-A- 0 538 153
- EP-A- 0 556 997
- EP-A- 0 613 667
- WO-A-91/04715
- FR-A- 2 521 421
- US-A- 3 806 961
- US-A- 4 151 615

## Description

L'invention concerne un implant trochléen pour prothèse fémoro-patellaire et son instrumentation de pose.

On a déjà développé des prothèses fémoro-patellaires du type non contraint dans lesquelles un implant rotulien à calotte sphérique est guidé sur une surface sensiblement torique d'un implant trochléen, ces prothèses permettant de réduire la résection osseuse au moment de leur pose et étant susceptibles d'être associées à des prothèses fémoro-tibiales mono ou bi-compartimentales, ou bien d'être remplacées par des prothèses totales du genou. Ces prothèses fémoro-patellaires sont fournies avec des ancillaires de pose plus ou moins sophistiqués, destinés à faciliter la tâche des chirurgiens orthopédistes et la pose de la prothèse.

L'invention a pour objet des perfectionnements à une prothèse fémoro-patellaire du type non contraint, visant notamment à améliorer la stabilité de la prothèse, à simplifier sa technique de pose, et à réduire l'importance de la résection osseuse du fémur tout en augmentant la précision du positionnement et de l'orientation de la prothèse.

L'invention propose à cet effet un implant trochléen pour prothèse fémoro-patellaire, cet implant comprenant une face antérieure incurvée concave de réception d'un élément rotulien et une face postérieure convexe d'application et de fixation sur l'extrémité distale du fémur, caractérisé en ce que la face postérieure de l'implant comporte une nervure longitudinale en saillie destinée à être emboîtée dans une rainure de forme correspondante usinée dans la trochlée.

Cette nervure longitudinale de l'implant trochléen facilite beaucoup la pose de l'implant, en déterminant son orientation de façon positive. Par ailleurs, elle augmente largement la stabilité de la prothèse.

Selon d'autres caractéristiques de l'invention, cette nervure longitudinale est à section transversale rectangulaire, l'implant a sensiblement la forme d'un L constitué d'une branche supérieure ou branche trochléenne et d'une branche inférieure ou branche intercondylienne, et ladite nervure s'étend sur toute la longueur de la branche supérieure ou branche trochléenne de l'implant.

Avantageusement, la face postérieure convexe de l'implant a une section transversale en arc de cercle dont le rayon est inférieur à celui de la section transversale de sa face antérieure, et les deux branches supérieure et inférieure de l'implant font entre elles un angle obtus d'environ 115° dans le plan sagittal.

Cette configuration des faces antérieure et postérieure de l'implant trochléen contribue également à faciliter sa pose qui peut être réalisée entièrement par fraisage, de façon précise et avec une résection osseuse minimale.

L'invention propose également une instrumentation de pose d'un implant trochléen du type précité, caractérisée en ce qu'elle comprend :
- un premier guide de fraisage de la face antérieure de la trochlée, associé à des moyens de positionnement sur les faces distales et postérieures des condyles et comprenant des moyens de guidage de l'axe d'une fraise parallèlement à l'axe de la trochlée,
- un deuxième guide de fraisage d'une rainure longitudinale dans la face antérieure de la trochlée, associé aux mêmes moyens de positionnement que précités et comprenant des moyens de guidage d'une fraise dans une direction perpendiculaire à l'axe de cette fraise, et
- un troisième guide de fraisage, de forme parallélépipèdique et comportant une nervure longitudinale destinée à être engagée dans la rainure fraisée de la trochlée et un alésage de guidage d'une fraise, l'axe de cet alésage étant oblique par rapport à l'axe de la nervure et formant avec celui-ci un angle égal à celui des faces postérieures des deux branches de l'implant trochléen.

Dans un mode de réalisation préféré de l'invention, les moyens de positionnement du premier et du deuxième guide de fraisage comprennent deux branches parallèles d'appui sur les faces postérieures des condyles, une traverse reliant entre elles ces deux branches et portant deux montants parallèles d'appui sur les faces distales des condyles, et un coulisseau guidé en translation entre les deux montants et comportant des moyens de guidage d'une tige destinée à être engagée dans le canal médullaire du fémur.

Les premier et deuxième guides de fraisage comprennent chacun deux glissières parallèles destinées à recevoir les montants précités des moyens de positionnement, et des moyens de blocage en position des glissières sur les montants.

Ces moyens permettent de déterminer l'axe de la trochlée, d'effectuer un fraisage de la face antérieure de la trochlée sur une longueur et une profondeur déterminées, de former ensuite une rainure de dimension déterminée dans la face fraisée de la trochlée, puis d'effectuer un fraisage de l'espace intercondylien dans une direction déterminée de façon positive, cette direction faisant avec l'axe de la rainure un angle qui correspond exactement à celui des deux branches de l'implant trochléen.

En raison de sa stabilité, l'implant trochléen selon l'invention peut être posé sans ciment, ce qui est favorable à une reprise ultérieure par une prothèse totale du genou.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue en élévation d'un implant trochléen selon l'invention ;
- la figure 2 est une vue de gauche de cet implant ;
- la figure 3 est une vue en coupe longitudinale selon la ligne III-III de la figure 2 ;
- la figure 4 est une vue en coupe transversale selon la ligne IV-IV de la figure 1 ;
- la figure 5 est une vue en perspective de l'implant ;
- la figure 6 illustre schématiquement les trois opérations d'usinage de l'extrémité distale du fémur pour la pose de l'implant ;
- les figures 7, 8 et 9 sont des vues en élévation, de côté et de dessus respectivement des moyens de positionnement des guides de fraisage ;
- les figures 10 et 11 sont des vues en coupe longitudinale et de dessus respectivement du guide de fraisage de la trochlée ;
- les figures 12 et 13 sont des vues en coupe longitudinale et de dessus respectivement du deuxième guide de fraisage ; et
- les figures 14 et 15 sont des vues en élévation et de dessous du troisième guide de fraisage.

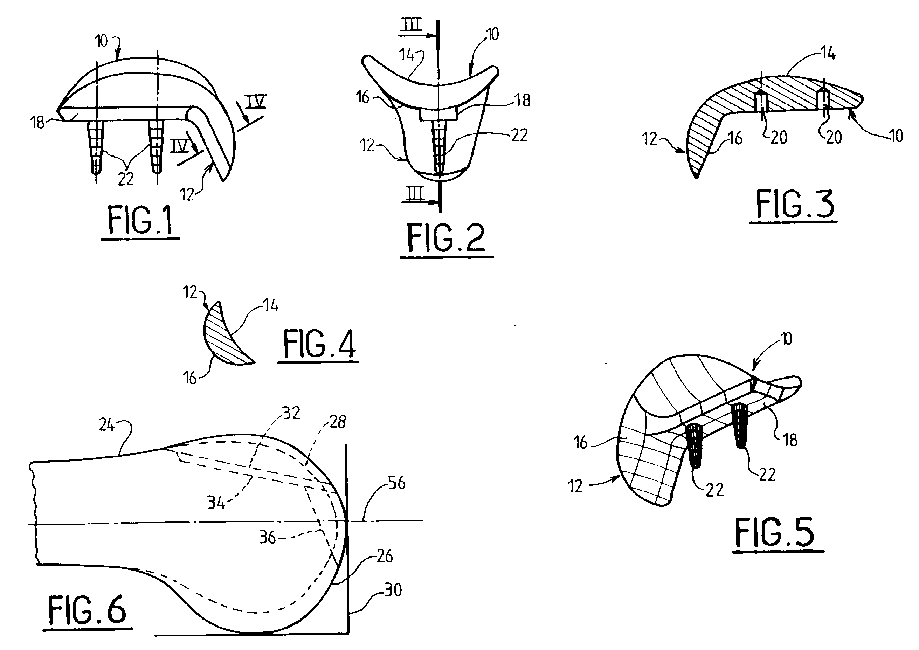

On se réfère d'abord aux figures 1 à 5 qui représentent l'implant trochléen selon l'invention.

Comme on le voit bien sur les figures 1 et 3, cet implant a sensiblement une forme de L à angle obtus, comprenant une branche supérieure 10 ou branche trochléenne et une branche inférieure 12 ou branche inter-condylienne.

La face antérieure 14 de l'implant est de forme concave en section transversale et convexe en section longitudinale et est destinée à recevoir un implant rotulien en forme de calotte sphérique. Pour cette raison, les sections transversales de la face antérieure 14 de l'implant trochléen sont constituées par des arcs de cercle.

La face postérieure 16 de l'implant trochléen est de forme convexe et sa section transversale est constituée d'un arc de cercle ayant un rayon inférieur à celui de la section transversale de la face antérieure de l'implant.

Comme on le voit bien en figure 2, le bord latéral externe de l'implant est plus relevé que le bord latéral interne, ce qui permet un meilleur guidage de l'implant rotulien et correspond mieux au modèle anatomique.

Selon l'invention, la face postérieure de l'implant comporte une nervure longitudinale 18 de forme rectangulaire, qui s'étend sur toute la longueur de la branche supérieure ou branche trochléenne 10 de l'implant. Deux trous borgnes 20 sont formés dans cette nervure 18 et débouchent perpendiculairement sur la face postérieure de celle-ci pour recevoir des pointes d'ancrage 22 du type en "sapin". En variante, l'implant peut être formé d'une pièce avec les pointes d'ancrage.

Le rayon de la section transversale de la face antérieure 14 de l'implant trochléen est constant sur toute la longueur de l'implant et correspond au rayon de la calotte sphérique de l'implant rotulien.

Le rayon de la section transversale de la face postérieure 16 de la première branche 10 de l'implant trochléen est constant et est par exemple de l'ordre de 25 mm, tandis que le rayon de la section transversale de la face postérieure 16 de la deuxième branche 12 de l'implant est constant et de l'ordre de 10 mm dans l'exemple représenté.

La pose de cet implant sur l'extrémité distale d'un fémur implique donc que l'on usine des surfaces en portion de cylindre dans la face antérieure de la trochlée et dans l'échancrure intercondylienne, sur lesquelles on appliquera respectivement la première branche 10 et la deuxième branche 12 de l'implant trochléen.

On a représenté schématiquement en figure 6 l'extrémité distale d'un fémur, la référence 24 désignant la face antérieure du fémur, la référence 26 désignant le contour d'un condyle et la référence 28 désignant le fond de l'échancrure intercondylienne vue de profil et représentée en trait pointillé en figure 6.

La pose de l'implant trochléen selon l'invention s'effectue de la façon générale suivante :
on commence par prendre une référence sur les condyles 26 au moyen d'une équerre double 30, puis on effectue un premier fraisage de la face antérieure de la trochlée, correspondant à la trace 32 représentée en pointillé, on forme par fraisage dans cette partie usinée de la trochlée une rainure longitudinale dont on a représenté la trace 34 en pointillé, puis on effectue dans l'échancrure intercondylienne un fraisage dont la trace 36 est également représentée en pointillé. Il suffit ensuite de percer dans le fond de la rainure 34 deux avant-trous destinés à recevoir les pointes d'ancrage 22 de l'implant, qui se trouve dans la position représentée en figure 1 quand on le pose sur l'extrémité distale du fémur dans la position de la figure 6.

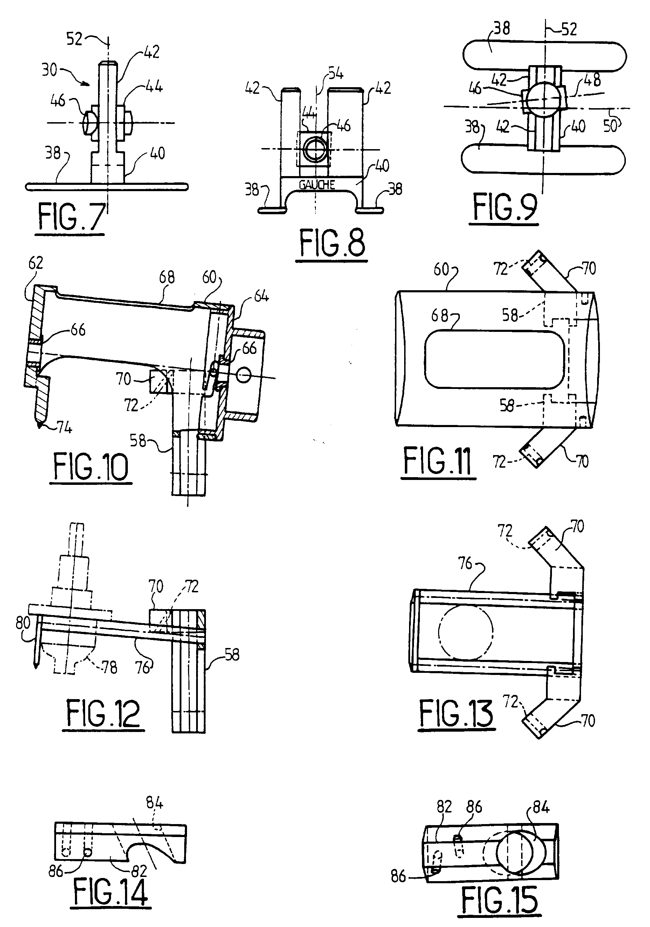

Les ancillaires de pose de l'implant selon l'invention ont été représentés en détail dans les figures 7 à 15.

On retrouve aux figures 7 à 9 l'équerre double 30 qui constitue des moyens de positionnement précis des deux guides de fraisage de la trochlée dont l'un est représenté aux figures 10 et 11 et l'autre aux figures 12 et 13.

L'équerre double 30 comprend deux branches ou pattes parallèles 38 destinées à être appliquées chacune sur la face postérieure d'un condyle 26, une traverse 40 reliant les deux branches ou pattes 38 entre elles, et deux montants parallèles 42 qui sont portés par la traverse 40 et qui s'étendent perpendiculairement aux branches ou pattes 38.

Un coulisseau 44 est guidé en translation entre les montants 42 et porte un manchon cylindrique 46 dont l'axe 48 s'étend dans un plan parallèle aux branches ou pattes 38 mais forme avec la direction longitudinale 50 de ces branches un angle de l'ordre de 6°, qui correspond à l'angle que l'on trouve normalement entre l'axe d'un fémur et l'axe de la trochlée.

Comme on le voit bien sur les figures 7 à 9, l'équerre double 30 est de forme symétrique par rapport à un plan transversal médian dont la trace est désignée par la référence 52 aux figures 7 et 9 et est dissymétrique par rapport à un plan sagittal médian dont la trace est désignée par la référence 54 en figure 8, de façon à ce que cette équerre double puisse être utilisée sur un fémur gauche quand elle est tournée d'un côté et sur un fémur droit quand elle est tournée de l'autre côté, les côtés gauche et droit étant par exemple repérés par gravure d'une indication correspondante comme on peut le voir en figure 8.

Le manchon 46 sert au guidage d'une tige cylindrique rectiligne destinée à être engagée dans le canal médullaire du fémur, que l'on a représenté schématiquement par un trait d'axe 56 en figure 6.

Lorsque l'équerre double 30 a été positionnée correctement sur l'extrémité distale d'un fémur (les branches 38 étant en appui sur les faces postérieures des condyles, les montants 42 étant appliqués sur la face distale des condyles et une tige cylindrique étant engagée dans le manchon 46 et dans le canal médullaire du fémur), on vient poser sur cette équerre double le guide de fraisage qui est représenté aux figures 10 et 11.

Ce guide de fraisage comprend deux glissières parallèles 58 qui s'engagent sur les montants 42 de l'équerre double 30 et qui supportent un carter semi-cylindrique 60 dont les deux extrémités axiales sont fermées par des plaques 62 et 64 comportant chacune une bague axiale 66 de guidage en rotation de l'axe d'une fraise non représentée. Un système de montage à baïonnette permet de fixer amoviblement la plaque 64 sur l'extrémité distale du carter semi-cylindrique 60.

Celui-ci est ouvert sur sa moitié inférieure, tandis que sa moitié supérieure comporte une lumière 68 permettant de suivre le déplacement de la fraise d'une extrémité à l'autre du carter.

Deux pattes latérales 70 portées par le carter 60 s'écartent en oblique de celui-ci et comportent à leur extrémité libre un trou 72 destiné à recevoir une pointe d'immobilisation du carter sur le fémur.

La plaque 62 fermant l'extrémité proximale du carter 60 comporte en partie inférieure une pointe 74 d'appui sur la face antérieure du fémur. Avantageusement, la distance entre cette pointe et l'axe du carter 60 est réglable.

L'angle que fait l'axe du carter 60 avec l'axe des glissières 58 dans le plan vertical est obtus, de l'ordre de 95°, ce qui permet de fraiser dans la face antérieure de la trochlée une cuvette cylindrique qui remonte légèrement en direction de l'extrémité proximale du fémur.

Le guide de fraisage représenté aux figures 10 et 11 est utilisé de la façon suivante :
- l'équerre double 30 étant en place sur l'extrémité distale du fémur, on engage les glissières 58 du guide de fraisage sur les montants 42 de l'équerre double et on règle la position en hauteur de ce guide au moyen d'un gabarit comprenant une tige cylindrique engagée dans les bagues 66 des extrémités du carter 60 et portant un disque dont le rayon est égal à celui de la fraise à utiliser diminué de la résection osseuse à pratiquer. Une vis de blocage (non représentée) prévue sur l'une des glissières 58 permet de fixer la position en hauteur du guide de fraisage sur l'équerre double et des pointes engagées dans les trous 72 des bras 70 permettent d'immobiliser le guide de fraisage sur le fémur.

Il faut alors démonter la plaque 64, retirer le gabarit, mettre à la place une fraise dont le diamètre correspond à la dimension de l'implant à poser, et remettre la plaque 64 en place, l'axe de la fraise étant alors guidé dans les bagues 66.

Par entraînement de la fraise en rotation et déplacement de cette fraise en translation à l'intérieur du carter 60 de son extrémité distale à son extrémité proximale, on peut creuser dans la face antérieure de la trochlée une cuvette cylindrique dont les dimensions correspondent à celles de la face postérieure de la première branche 10 de l'implant trochléen.

On démonte ensuite le guide de fraisage des figures 10 et 11 en laissant en place l'équerre double 30, et on pose sur cette équerre double le guide de fraisage représenté aux figures 12 et 13, qui va permettre de former dans la cuvette précitée une rainure longitudinale de réception de la nervure 18 de l'implant trochléen.

Le guide de fraisage représenté aux figures 12 et 13 comprend, comme celui des figures 10 et 11, deux glissières 58 destinées à s'engager sur les montants 42 de l'équerre double, et deux pattes latérales 70 dont les extrémités libres comportent des trous 72 de passage de pointes d'immobilisation sur le fémur.

L'extrémité supérieure des glissières 58 est solidaire d'un cadre rectangulaire 76 dont le plan fait un angle obtus de l'ordre de 95° avec l'axe des glissières 58, ce cadre 76 servant de guidage à une fraise 78 représentée en trait fantôme en figure 12 et dont l'axe est perpendiculaire au plan du cadre 76. L'extrémité inférieure cylindrique de la fraise 78 a un diamètre qui correspond à la largeur de la rainure à creuser, c'est-à-dire à la largeur de la nervure 18 de l'implant trochléen.

L'extrémité distale du cadre 76 comporte une plaque 80 se terminant en partie inférieure par une pointe d'appui sur la face antérieure du fémur, la distance entre cette pointe et le cadre 76 étant de préférence réglable.

Ce deuxième guide de fraisage est utilisé de la façon suivante :
on engage ces glissières 58 sur les montants 42 de l'équerre double, on le positionne en hauteur au moyen d'un gabarit placé sur le cadre 76 et dont les dimensions sont fonction de celles de l'implant à poser et des dimensions de la fraise 78 utilisée, on immobilise ce guide de fraisage sur l'équerre double au moyen d'une vis de blocage traversant une glissière 58, on engage deux pointes dans les trous 72 des pattes latérales 70, on dispose la fraise 78 dans le cadre 76 comme représenté en figure 12 et on la déplace d'une extrémité à l'autre de ce cadre pour creuser une rainure longitudinale dans la cuvette cylindrique préalablement usinée dans la face antérieure de la trochlée.

On peut ensuite démonter ce deuxième guide de fraisage, on retire l'équerre double 30 et on place dans la rainure que l'on vient de creuser le troisième guide de fraisage représenté aux figures 14 et 15.

Ce troisième guide de fraisage est de forme générale parallélépipèdique rectangle et comporte sur une de ses faces une nervure rectangulaire 82 dont la forme correspond à celle de la nervure 18 de l'implant trochléen. Un alésage 84 est formé en oblique dans le corps parallélépipèdique et dans la nervure 82, l'axe de cet alésage faisant avec l'axe longitudinal du corps un angle de l'ordre de 115°, égal à l'angle entre les deux branches de l'implant trochléen.

On utilise ce troisième guide de fraisage de la façon suivante :
sa nervure 82 est engagée dans la rainure que l'on vient de creuser dans la face antérieure de la trochlée, et ce troisième guide de fraisage est positionné longitudinalement dans la rainure de façon à ce que son alésage 84 débouche dans l'échancrure intercondylienne du fémur. Un gabarit formé d'une tige cylindrique dont une partie est au diamètre de l'alésage 84 et dont l'autre partie est de diamètre inférieur, la différence des rayons étant égale à la résection osseuse à pratiquer, est engagé dans l'alésage 84 pour parfaire le positionnement longitudinal de ce guide de fraisage. Des pointes engagées dans des trous obliques 86 du corps permettent d'immobiliser ce guide sur le fémur, puis une fraise engagée dans l'alésage 84 permet de creuser dans l'échancrure intercondylienne une cuvette cylindrique aux dimensions de la face postérieure de la deuxième branche 12 de l'implant trochléen à poser.

On retire ensuite ce troisième guide de fraisage, on pose sur les parties usinées du fémur une prothèse d'essai ou prothèse fantôme comprenant des orifices à la place des pointes d'ancrage 22, on perce dans le fond de la rainure usinée dans la trochlée des avant-trous de réception de ces pointes d'ancrage, puis on pose l'implant trochléen représenté aux figures 1 à 5, en enfonçant à force ces pions d'ancrage 22 dans les avant-trous que l'on vient de former.

Cet implant trochléen peut être posé sans ciment. Il est avantageux dans ce cas que sa face postérieure, en contact avec l'os, comporte un revêtement poreux et/ou un revêtement d'hydroxyapatite ou de toute autre matière facilitant la réhabilitation osseuse.

## Revendications

1. Implant trochléen pour prothèse fémoro-patellaire, cet implant comprenant une face antérieure (14) incurvée concave de réception d'un élément rotulien et une face postérieure convexe (16) d'application et de fixation sur l'extrémité distale du fémur, caractérisé en ce que la face postérieure (16) de l'implant comprend une nervure longitudinale (18) en saillie destinée à être emboîtée dans une rainure de forme correspondante usinée dans la trochlée.

2. Implant selon la revendication 1, caractérisé en ce que ladite nervure longitudinale (18) est à section transversale rectangulaire.

3. Implant selon la revendication 1 ou 2, caractérisé en ce qu'il a sensiblement la forme d'un L comprenant une branche supérieure (10) ou branche trochléenne et une branche inférieure (12) ou branche inter-condylienne.

4. Implant selon la revendication 3, caractérisé en ce que ladite nervure longitudinale (18) s'étend sur toute la longueur de la branche supérieure (10) ou branche trochléenne de l'implant.

5. Implant selon l'une des revendications précédentes, caractérisé en ce qu'il comprend des pointes d'ancrage (22) sur la nervure (18) précitée s'étendant perpendiculairement à celle-ci.

6. Implant selon l'une des revendications 1 à 5, caractérisé en ce que sa face postérieure convexe (16) a une section transversale en arc de cercle dont le rayon est inférieur à celui de la section transversale de sa face antérieure (14).

7. Implant selon l'une des revendications 3 à 6, caractérisé en ce que ses deux branches supérieure (10) et inférieure (12) font entre elles un angle obtus d'environ 115° dans le plan sagittal.

8. Implant selon l'une des revendications 3 à 7, caractérisé en ce que le bord latéral externe de la branche supérieure (10) est plus élevé que le bord latéral interne de cette branche.

9. Instrumentation de pose d'un implant trochléen selon l'une des revendications précédentes, caractérisé en ce qu'elle comprend :
- un premier guide (60,62,64) de fraisage de la face antérieure de la trochlée, associé à des moyens (30) de positionnement sur les faces distales et postérieures des condyles et comprenant des moyens (66) de guidage de l'axe d'une fraise parallèlement à l'axe de la trochlée,
- un deuxième guide (76) de fraisage d'une rainure longitudinale dans la face antérieure de la trochlée, associé aux mêmes moyens de positionnement (30) et comprenant des moyens (76) de guidage d'une fraise (78) dans une direction perpendiculaire à l'axe de cette fraise, et
- un troisième guide de fraisage, de forme parallélépipèdique et comportant une nervure longitudinale (82) destinée à être engagée dans la rainure fraisée de la trochlée et un alésage (84) de guidage d'une fraise, l'axe de cet alésage étant oblique par rapport à l'axe de la nervure (82) et formant avec celui-ci un angle égal à celui des faces postérieures des deux branches (10,12) de l'implant trochléen.

10. Instrumentation selon la revendication 9, caractérisé en ce que les moyens (30) de positionnement des premier et deuxième guides de fraisage comprennent deux branches parallèles (38) d'appui sur les faces postérieures des condyles, une traverse (40) reliant entre elles ces deux branches et portant deux montants parallèles (42) d'appui sur les faces distales des condyles, et un coulisseau (44) guidé en translation entre les deux montants (42) et comportant des moyens (46) de guidage d'une tige destinée à être engagée dans le canal médullaire du fémur.

11. Instrumentation selon la revendication 10, caractérisée en ce que les premier et deuxième guides de fraisage comprennent chacun deux glissières parallèles (58) destinées à recevoir les montants (42) précités des moyens de positionnement (30), et des moyens de blocage en position des glissières sur les montants.

## Claims

1. A trochlea implant for a femoro-patellar prosthesis, the implant comprising a concave curved anterior face (14) for receiving a patellar element and a convex posterior face (16) for applying against and fixing to the distal end of the femur, the implant being characterized in that its posterior face (16) includes a projecting longitudinal rib (18) designed to be engaged in a groove of corresponding shape machined in the trochlea.

2. An implant according to claim 1, characterized in that said longitudinal rib (18) is of rectangular cross-section.

3. An implant according to claim 1 or 2, characterized in that it is substantially L-shaped, comprising an upper or "trochlea" branch (10) and a lower or "inter-condyle" branch (12).

4. An implant according to claim 3, characterized in that said longitudinal rib (18) extends over the entire length of the upper or "trochlea" branch (10) of the implant.

5. An implant according to any preceding claim, characterized in that it includes anchor pegs (22) on the above-specified rib (18) and extending perpendicularly thereto.

6. An implant according to any one of claims 1 to 5, characterized in that its convex posterior face (16) has a cross-section in the form of a circular arc of radius less than that of the cross-section of its anterior face (14).

7. An implant according to any one of claims 3 to 6, characterized in that its upper and lower branches (10, 12) are at an obtuse angle to each other in the sagittal plane, which angle is equal to about 115°.

8. An implant according to any one of claims 3 to 7, characterized in that the lateral side edge of the upper branch (10) is higher than the medial side edge of said branch.

9. Instrumentation for implanting a trochlea implant according to any preceding claim, characterized in that it comprises:
• a first milling guide (60, 62, 64) for milling the anterior face of the trochlea, associated with positioning means (30) for positioning on the distal and posterior faces of the condyles and including guide means (66) for guiding the axis of a milling head parallel to the axis of the trochlea;
• a second milling guide (76) for milling a longitudinal groove in the anterior face of the trochlea, associated with the same positioning means (30) and including guide means (76) for guiding a milling head (78) in a direction perpendicular to the axis of said milling head; and
• a third milling guide that is in the form of a rectangular parallelepiped, including a longitudinal rib (82) designed to be engaged in the groove milled in the trochlea, and a milling head guide bore (84), with the axis of said bore being oblique relative to the axis of the rib (82) and forming an angle therewith which is equal to the angle between the posterior faces of the two branches (10,12) of the trochlea implant.

10. Instrumentation according to claim 9, characterized in that the positioning means (30) for positioning the first and second milling guides comprises two parallel branches (38) for bearing against the posterior faces of the condyles, a crossbar (40) interconnecting said two branches and carrying two parallel uprights (42) for bearing against the distal faces of the condyles, and a slider (44) guided in translation between the two uprights (42) and including means (46) for guiding a rod that is designed to be engaged in the medullary canal of the femur.

11. Instrumentation according to claim 10, characterized in that each of the first and second milling guides comprises two parallel slideways (58) designed to receive the above-specified uprights (42) of the positioning means (30), and means for locking the slideways in position on the uprights.

## Patentansprüche

1. Trochleärimplantat für eine Femoral-Patellar-Prothese, wobei dieses Implantat eine gekrümmte konkave Vorderseite (14) für die Aufnahme eines Kniescheibenelements und eine konvexe Hinterseite (16) für das Aufbringen und die Befestigung auf dem distalen Ende des Femurs hat, dadurch **gekennzeichnet,** daß die Hinterseite (16) des Implantats eine vorspringende Längsrippe (18) enthält, die dazu bestimmt ist, in eine Nut von entsprechender Form eingefügt zu werden, weiche in der Trochlea hergestellt ist.

2. Implantat gemäß Anspruch 1, dadurch **gekennzeichnet,** daß die Längsrippe (18) von rechteckigem Querschnitt ist.

3. Implantat gemäß Anspruch 1 oder 2, dadurch **gekennzeichnet,** daß es im wesentlichen die Form eines L hat, welches einen oberen Schenkel (10) oder Trochleärschenkel und einen unteren Schenkel (12) oder Interkondylärschenkel hat.

4. Implantat gemäß Anspruch 3, dadurch **gekennzeichnet,** daß sich die Längsrippe (18) über die gesamte Länge des oberen Schenkels (10) oder Trochleärschenkel des Implantats erstreckt.

5. Implantat gemäß einem der vorhergehenden Ansprüche, dadurch **gekennzeichnet,** daß es Verankerungsstifte bzw. -nasen bzw. -spitzen (22) auf der Rippe (18) umfaßt, die sich senkrecht zu derselben erstrecken.

6. Implantat gemäß einem der Ansprüche 1 bis 5, dadurch **gekennzeichnet,** daß seine konvexe Hinterseite (16) einen kreisbogenförmigen Querschnitt hat, dessen Radius kleiner als jener des Querschnitts der Vorderseite (14) ist.

7. Implantat gemäß einem der Ansprüche 3 bis 6, dadurch **gekennzeichnet,** daß seine beiden Schenkel, oberer (10) und unterer (12), miteinander einen stumpfen Winkel von ungefähr 115° in der Sagittalebene bilden.

8. Implantat gemäß einem der Ansprüche 3 bis 7, dadurch **gekennzeichnet,** daß der äußere Seitenrand des oberen Schenkels (10) stärker erhöht ist als der innere Seitenrand dieses Schenkels.

9. Instrumentierung zum Einsetzen eines Trochleärimplantats gemäß einem der vorhergehenden Ansprüche, dadurch **gekennzeichnet,** daß sie folgendes umfaßt:
- eine erste Führung (60, 62, 64) zum Fräsen der Vorderseite der Trochlea, die mit Mitteln (30) für die Positionierung auf den distalen und hinteren Seiten der Kondylen verbunden ist und Mittel (66) für die Führung der Achse einer Fräse parallel zu der Achse der Trochlea umfaßt,
- eine zweite Führung (76) zum Fräsen einer Längsnut in die Vorderseite der Trochlea, verbunden mit den gleichen Mitteln für die Positionierung (30) und umfassend Mittel (76) für die Führung einer Fräse (78) in einer Richtung senkrecht zu der Achse dieser Fräse, und
- eine dritte Führung zum Fräsen, von parallelepipedischer Form und enthaltend eine Längsrippe (82), welche dazu bestimmt ist, in die gefräste Nut der Trochlea eingefügt zu werden, und eine Bohrung (84) für die Führung einer Fräse, wobei die Achse dieser Bohrung geneigt mit Bezug auf die Achse der Rippe (82) ist und mit derselben einen Winkel bildet, der gleich demjenigen der Hinterseiten der beiden Schenkel (10, 12) des Trochleärimplantats ist.

10. Instrumentierung gemäß Anspruch 9, dadurch **gekennzeichnet,** daß die Mittel (30) für die Positionierung der ersten und zweiten Führung zum Fräsen zwei parallele Schenkel (38) für die Anlage auf den Hinterseiten der Kondylen, eine Strebe bzw. Traverse (40), welche diese beiden Schenkel miteinander verbindet und zwei parallele Säulen (42) für die Anlage auf den distalen Seiten der Kondylen trägt, sowie eine Kulisse (44), die in Translation zwischen den beiden Säulen (42) geführt ist und Mittel (46) für die Führung eines Schafts hat, der dazu bestimmt ist, in den Medullärkanal des Femurs eingefügt zu werden.

11. Instrumentierung gemäß Anspruch 10, dadurch **gekennzeichnet,** daß die erste und zweite Führung zum Fräsen je zwei parallele Gleitbahnen bzw. Rinnen bzw. Führungsbahnen (58) umfaßt, die dazu bestimmt sind, die vorgenannten Säulen (42) der Positionierungsmittel (30) aufzunehmen, und Mittel für die Blockierung bzw. Arretierung der Gleitbahnen bzw. Rinnen bzw. Führungsbahnen in Position auf den Säulen.
